# EUROPEAN PATENT APPLICATION

(11) **EP 4 800 025 A1**
(43) Date of publication of application: **02.09.2026**
(21) Application number: 24902717.8
(22) Date of filing: 06.12.2024
(51) Int. Cl.: C07K 14/08, C12N 15/81, A61K 39/12, A61K 39/39

(54) **HEXAVALENT NOROVIRUS IMMUNE COMPOSITION, KIT THEREOF, AND USE**

(30) Priority: 14.12.2023 CN 202311729614
(71) Applicant: Grand Theravac Life Science (Nanjing) Co., Ltd., Nanjing, Jiangsu 210032 (CN)
(72) Inventor: LI, Jianqiang, Nanjing, Jiangsu 210032 (CN); GE, Jun, Nanjing, Jiangsu 210032 (CN); ZHOU, Tong, Nanjing, Jiangsu 210032 (CN); GU, Yue, Nanjing, Jiangsu 210032 (CN); WANG, Xiaodong, Nanjing, Jiangsu 210032 (CN); CHEN, Xiaoxiao, Nanjing, Jiangsu 210032 (CN); SHI, Yinghui, Nanjing, Jiangsu 210032 (CN); HUANG, Hongying, Nanjing, Jiangsu 210032 (CN); LI, Ling, Nanjing, Jiangsu 210032 (CN); LU, Jinbiao, Nanjing, Jiangsu 210032 (CN); LI, Chunming, Nanjing, Jiangsu 210032 (CN)
(74) Representative: Uexküll & Stolberg
(86) International application number: PCT/CN2024/137327
(87) International publication number: WO 2025/124296

(57) **Abstract**

Provided is a hexavalent norovirus immunogenic composition, comprising virus-like particles of norovirus genotypes GI.1, GII.2, GII.3, GII.4, GII.6, and GII.17, or active fragments thereof. Also provided is a kit comprising the hexavalent norovirus immunogenic composition. Also provided are uses of the hexavalent norovirus immunogenic composition and the kit. The composition or the kit is capable of achieving effective immunization against the six norovirus genotypes GI.1, GII.2, GII.3, GII.4, GII.6, and GII.17, and providing long-lasting efficacy after administration.

## Description

### Cross Reference to Related Application

This application claims priority to Chinese Patent Application No. 202311729614.9, filed on December 14, 2023, the entire content of which is incorporated herein by reference.

### Technical Field

The present invention belongs to the technical field of biopharmaceuticals and relates to a hexavalent norovirus immunogenic composition and a kit comprising the composition. The present invention also provides uses of the composition and the kit.

### Background Art

Human norovirus was first identified in 1972. It is a major pathogen responsible for acute viral gastroenteritis, featuring high contagiousness and a tendency to cause localized outbreaks, and is predominantly transmitted via the fecal-oral route. The primary symptoms of infection are vomiting and diarrhea, followed by nausea, abdominal pain, headache, fever, chills, and myalgia. Severe infections may result in dehydration and even death.

Human norovirus is a non-enveloped, single-stranded, positive-sense RNA virus, belonging to the family Caliciviridae. It has a diameter of 26-35 nm, is non-enveloped, rough-surfaced, and spherical, and exhibits icosahedral symmetry. It was isolated from the feces of a patient with acute gastroenteritis. The full-length genome of norovirus is approximately 7.7 kb and contains three Open Reading Frames (ORFs).

Noroviruses are classified into seven genotypes based on the amino acid sequence of VP1 and further subdivided into more than 30 subgenotypes. Among them, GI, GII, and GVI are closely associated with human diseases, with GII genotypes being the most common, followed by GI genotypes. Epidemiological studies have shown that in China, GII.3, GII.4, and GII.17 are the most prevalent among the GII genotypes, while GI.1 is the more common among the GI genotypes.

With respect to norovirus vaccines, most research institutions worldwide are currently focused on developing monovalent and bivalent norovirus vaccines. However, combined immunization with antigens of multiple genotypes requires consideration of mutual interference among different genotypes. For example, previous studies on the trivalent poliovirus vaccine and the tetravalent dengue vaccine have reported mutual interference among different genotypes (Xiong Pei, Immunological Evaluation of Tetravalent Norovirus Vaccine and Identification of Blocking Antibody Epitopes for GI.14 Norovirus, Master's Thesis, 2019: 36-37).

Therefore, the development of a multivalent norovirus vaccine with an anti-interference capability and a favorable immune effect is an urgent problem to be solved at present, and also a major challenge in the field of multivalent vaccines.

### Summary of the Invention

In view of the deficiencies of the prior art, it is therefore an objective of the present invention to provide an immunogenic composition against noroviruses of multiple genotypes, which covers norovirus genotypes GI.1, GII.2, GII.3, GII.4, GII.6, and GII.17. The present invention also provides a kit comprising the immunogenic composition. The present invention also provides uses of the immunogenic composition and the kit.

The objectives of the present invention are achieved by the following technical solutions.

In an aspect, the present invention provides a hexavalent norovirus immunogenic composition, comprising virus-like particles of norovirus genotypes GI.1, GII.2, GII.3, GII.4, GII.6, and GII.17, or active fragments thereof.

In the hexavalent norovirus immunogenic composition according to the present invention, the weight ratio of the virus-like particles of norovirus genotypes GI.1, GII.2, GII.3, GII.4, GII.6, and GII.17 or active fragments thereof is 40: 20: (10~100): 40: 40: 40, preferably 40: 20: (30~40): 40: 40: 40.

In another aspect, the present invention also provides an immunoassay kit comprising the hexavalent norovirus immunogenic composition according to the present invention.

Preferably, the immunoassay kit is a kit for detecting norovirus.

In a further aspect, the present invention also provides use of the hexavalent norovirus immunogenic composition according to the present invention or the immunoassay kit according to the present invention in the manufacture of the following products:
(1) a medicament for preventing and/or treating norovirus infection;
(2) a kit for diagnosing norovirus infection; or
(3) an immunogen for developing norovirus antibodies.

Preferably, the norovirus infection is gastroenteritis; more preferably, the norovirus infection is acute viral gastroenteritis.

As can be seen from the above technical solutions, the hexavalent norovirus immunogenic composition of the present invention, as well as the kit and the use thereof have at least the following beneficial effects:
The hexavalent norovirus immunogenic composition of the present invention provides effective immunization against the six norovirus genotypes GI.1, GII.2, GII.3, GII.4, GII.6, and GII.17. The experimental studies conducted by the inventors demonstrate that, the hexavalent norovirus immunogenic composition of the present invention is capable of maintaining a high level of IgG antibody for 8 weeks after three administrations.

In addition, the inventors have found through research that combining virus-like particles of norovirus genotypes GII.2, GII.4, GII.6, and GII.17 with those of norovirus genotypes GI.1 and GII.3 to form a hexavalent norovirus immunogenic composition improves the compatibility among various antigen genotypes, that is, it can significantly reduce the interference among different genotypes.

### Brief Description of the Drawings

FIG. 1 shows the SDS-PAGE patterns of the purified virus-like particles of various genotypes from Step (2) of Example 1 of the present application.
FIG. 2 shows the detection results of serum IgG antibody levels in mice for the hexavalent formulation group and the comparative sample group at Week 14 (wk14) in Experimental Example 1 of the present application.
FIG. 3 shows the detection results of serum IgG antibody levels in mice for the hexavalent formulation group and the monovalent formulation group at Week 14 (wk14) in Experimental Example 1 of the present application.

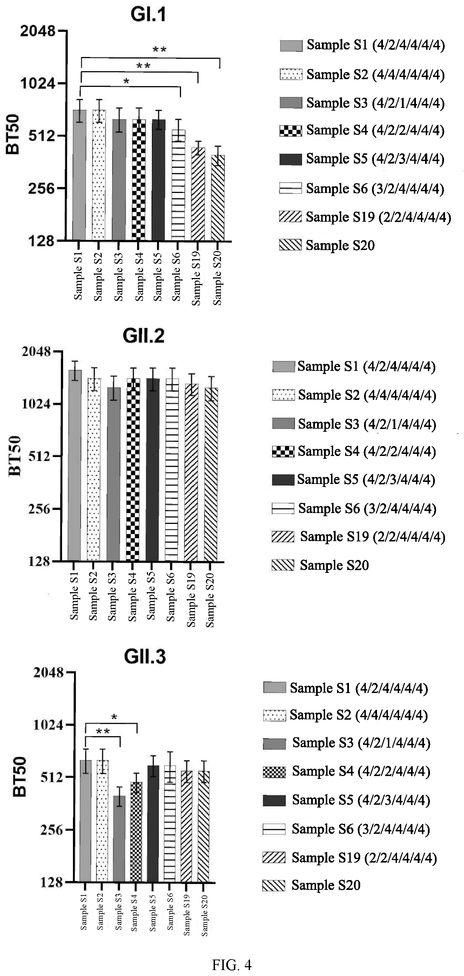

FIG. 4 shows the detection results of serum GMBT50 values for the hexavalent formulation group at different weight ratios at Week 8 (wk08) in Experimental Example 2 of the present application.

### Best Modes of Carrying Out the Invention

The present invention will be further described below with reference to the accompanying drawings and specific embodiments, which are not intended to limit the present invention. Various modifications or improvements may be made by those skilled in the art based on the basic concept of the present invention, and all such modifications and improvements shall fall within the scope of the present invention, provided that they do not depart from the basic concept thereof.

### Definitions

Unless defined otherwise, all scientific and technical terms used herein have the same meanings as understood by those of ordinary skill in the art. With regard to definitions and terms in the art, those skilled in the art may refer specifically to *Current Protocols in Molecular Biology* (Ausubel). The abbreviations for amino acid residues are the standard 3-letter and/or 1-letter codes used in the art to denote one of the 20 common L-amino acids.

In addition, it should be noted that, as used in this specification, the term "or" may be used interchangeably with the term "and/or", unless the context clearly indicates otherwise.

The terms "pharmaceutical composition", "combined drug", and "drug combination" used herein are interchangeable and refer to a combination of at least one drug and optional a pharmaceutically acceptable excipient or auxiliary material, which are put together to achieve a specific purpose. In certain embodiments, the composition encompasses components that are separated in time and/or space, provided that they can act together to achieve the objectives of the present invention. For example, the components contained in the pharmaceutical composition (e.g., GI.1, GII.2, GII.3, GII.4, GII.6, GII.17, or Al(OH)₃) may be administered to a subject either integrally or separately. When the components contained in the pharmaceutical composition are administered to a subject separately, they may be administered to the subject simultaneously or sequentially.

The term "therapeutically effective amount" or "effective amount" used herein refers to a dosage sufficient to demonstrate beneficial effects on the subject to whom it is administered. The actual dosage to be administered, as well as the administration rate and schedule will depend on the personal condition and severity of illness of the treated subject. The prescription for treatment (e.g., dosage determination, etc.) is ultimately the responsibility of general practitioners and other physicians and depends on their decisions, which often take into account the disease to be treated, the individual condition of the patient, the delivery site, the administration route, and other factors known to physicians.

The term "carrier" used herein refers to a substance that is mixed with a pharmaceutically active substance to form a dosage form for administration to a patient. It will neither cause significant irritation to the patient and nor eliminate the activity of the active pharmaceutical ingredient. Preferred pharmaceutical carriers are, in particular, water, buffered aqueous solutions, preferably isotonic saline solutions such as PBS (phosphate-buffered saline solutions), glucose, mannitol, dextrose, lactose, starch, magnesium stearate, cellulose, magnesium carbonate, 0.3% glycerol, hyaluronic acid, ethanol, or polyalkylene glycols such as polypropylene glycol, and triglycerides etc. The type of pharmaceutical carrier used depends in particular on whether the composition according to the invention is formulated for oral, nasal, intradermal, subcutaneous, intramuscular, or intravenous administration.

### Hexavalent Norovirus Immunogenic composition

The present invention provides a hexavalent norovirus immunogenic composition, comprising virus-like particles of norovirus genotypes GI.1, GII.2, GII.3, GII.4, GII.6, and GII.17, or active fragments thereof.

In the hexavalent norovirus immunogenic composition according to the present invention, the virus-like particle of norovirus genotype GI.1 or active fragment thereof comprises or consists of an amino acid sequence selected from the group consisting of:
(1) an amino acid sequence as set forth in SEQ ID NO: 1;
(2) an amino acid sequence having at least 90%, 91%, 92%, 93%, 94%, 95%, 96%, 97%, 98%, 99%, or more identity to the amino acid sequence as set forth in SEQ ID NO: 1; and
(3) an amino acid sequence having one or more, such as 1, 2, 3, 4, or 5 amino acid substitutions, deletions, or insertions relative to the amino acid sequence as set forth in SEQ ID NO: 1.

Preferably, the amino acid sequence of the virus-like particle of norovirus genotype GI.1 or active fragment thereof is as set forth in SEQ ID NO: 1.

In the hexavalent norovirus immunogenic composition according to the present invention, the virus-like particle of norovirus genotype GII.2 or active fragment thereof comprises or consists of an amino acid sequence selected from the group consisting of:
(1) an amino acid sequence as set forth in SEQ ID NO: 2;
(2) an amino acid sequence having at least 90%, 91%, 92%, 93%, 94%, 95%, 96%, 97%, 98%, 99%, or more identity to the amino acid sequence as set forth in SEQ ID NO: 2; and
(3) an amino acid sequence having one or more, such as 1, 2, 3, 4, or 5 amino acid substitutions, deletions, or insertions relative to the amino acid sequence as set forth in SEQ ID NO: 2.

Preferably, the amino acid sequence of the virus-like particle of norovirus genotype GII.2 or active fragment thereof is as set forth in SEQ ID NO: 2.

In the hexavalent norovirus immunogenic composition according to the present invention, the virus-like particle of norovirus genotype GII.3 or active fragment thereof comprises or consists of an amino acid sequence selected from the group consisting of:
(1) an amino acid sequence as set forth in SEQ ID NO: 3;
(2) an amino acid sequence having at least 90%, 91%, 92%, 93%, 94%, 95%, 96%, 97%, 98%, 99%, or more identity to the amino acid sequence as set forth in SEQ ID NO: 3; and
(3) an amino acid sequence having one or more, such as 1, 2, 3, 4, or 5 amino acid substitutions, deletions, or insertions relative to the amino acid sequence as set forth in SEQ ID NO: 3.

Preferably, the amino acid sequence of the virus-like particle of norovirus genotype GII.3 or active fragment thereof is as set forth in SEQ ID NO: 3.

In the hexavalent norovirus immunogenic composition according to the present invention, the virus-like particle of norovirus genotype GII.4 or active fragment thereof comprises or consists of an amino acid sequence selected from the group consisting of:
(1) an amino acid sequence as set forth in SEQ ID NO: 4;
(2) an amino acid sequence having at least 90%, 91%, 92%, 93%, 94%, 95%, 96%, 97%, 98%, 99%, or more identity to the amino acid sequence as set forth in SEQ ID NO: 4; and
(3) an amino acid sequence having one or more, such as 1, 2, 3, 4, or 5 amino acid substitutions, deletions, or insertions relative to the amino acid sequence as set forth in SEQ ID NO: 4.

Preferably, the amino acid sequence of the virus-like particle of norovirus genotype GII.4 or active fragment thereof is as set forth in SEQ ID NO: 4.

In the hexavalent norovirus immunogenic composition according to the present invention, the virus-like particle of norovirus genotype GII.6 or active fragment thereof comprises or consists of an amino acid sequence selected from the group consisting of:
(1) an amino acid sequence as set forth in SEQ ID NO: 5;
(2) an amino acid sequence having at least 90%, 91%, 92%, 93%, 94%, 95%, 96%, 97%, 98%, 99%, or more identity to the amino acid sequence as set forth in SEQ ID NO: 5; and
(3) an amino acid sequence having one or more, such as 1, 2, 3, 4, or 5 amino acid substitutions, deletions, or insertions relative to the amino acid sequence as set forth in SEQ ID NO: 5.

Preferably, the amino acid sequence of the virus-like particle of norovirus genotype GII.6 or active fragment thereof is as set forth in SEQ ID NO: 5.

In the hexavalent norovirus immunogenic composition according to the present invention, the virus-like particle of norovirus genotype GII.17 or active fragment thereof comprises or consists of an amino acid sequence selected from the group consisting of:
(1) an amino acid sequence as set forth in SEQ ID NO: 6;
(2) an amino acid sequence having at least 90%, 91%, 92%, 93%, 94%, 95%, 96%, 97%, 98%, 99%, or more identity to the amino acid sequence as set forth in SEQ ID NO: 6; and
(3) an amino acid sequence having one or more, such as 1, 2, 3, 4, or 5 amino acid substitutions, deletions, or insertions relative to the amino acid sequence as set forth in SEQ ID NO: 6.

Preferably, the amino acid sequence of the virus-like particle of norovirus genotype GII.17 or active fragment thereof is as set forth in SEQ ID NO: 6.

In the hexavalent norovirus immunogenic composition according to the present invention, the weight ratio of the virus-like particles of norovirus genotypes GI.1, GII.2, GII.3, GII.4, GII.6, and GII.17 or active fragments thereof is 40: 20: (10~100): 40: 40: 40, preferably 40: 20: (30~40): 40: 40: 40.

As a preferred embodiment, in the hexavalent norovirus immunogenic composition of the present invention, the amino acid sequence of the virus-like particle of norovirus genotype GI.1 or active fragment thereof is as set forth in SEQ ID NO: 1; the amino acid sequence of the virus-like particle of norovirus genotype GII.2 or active fragment thereof is as set forth in SEQ ID NO: 2; the amino acid sequence of the virus-like particle of norovirus genotype GII.3 or active fragment thereof is as set forth in SEQ ID NO: 3; the amino acid sequence of the virus-like particle of norovirus genotype GII.4 or active fragment thereof is as set forth in SEQ ID NO: 4; the amino acid sequence of the virus-like particle of norovirus genotype GII.6 or active fragment thereof is as set forth in SEQ ID NO: 5; the amino acid sequence of the virus-like particle of norovirus genotype GII.17 or active fragment thereof is as set forth in SEQ ID NO: 6; and the weight ratio of the virus-like particles of norovirus genotypes GI.1, GII.2, GII.3, GII.4, GII.6, and GII.17 or active fragments thereof is 40: 20: 40: 40: 40: 40.

To enhance the immune response and achieve optimal protective immunity in humans, the hexavalent norovirus immunogenic composition of the present invention may further comprise an adjuvant, preferably an aluminum adjuvant, such as aluminum hydroxide, aluminum phosphate, or aluminum sulfate, etc.

To maintain water and electrolyte balance, the hexavalent norovirus immunogenic composition of the present invention may further comprise sodium chloride.

The hexavalent norovirus immunogenic composition according to the present invention may be administered via any suitable routes, for example, orally, nasally, intradermally, subcutaneously, intramuscularly, or intravenously. Accordingly, the hexavalent norovirus immunogenic composition of the present invention may further comprise a carrier, such as water. In addition, if desired, the hexavalent norovirus immunogenic composition of the present invention may further comprise additives, such as wetting agents, emulsifiers, or buffer substances, etc.

In the hexavalent norovirus immunogenic composition of the present invention, the amounts of the aluminum adjuvant, sodium chloride, water, and additives, etc. may be determined with reference to the relevant solutions in the prior art, and the present invention is not limited thereto.

### Preparation Method of Hexavalent Norovirus Immunogenic Composition

The method for preparing the hexavalent norovirus immunogenic composition of the present invention comprises: preparation of recombinant strains expressing norovirus antigens and expression of recombinant proteins, purification of norovirus antigen proteins, mixing of norovirus antigens, and mixing and adsorption of norovirus antigen mixture solution with an adjuvant.

As a preferred embodiment, the method for preparing the hexavalent norovirus immunogenic composition of the present invention comprises the following steps:
(1) Preparation of recombinant strains expressing norovirus antigens and expression of recombinant proteins

Codon optimization is performed on the gene sequences corresponding to the virus-like particle proteins of norovirus genotypes GI.1, GII.2, GII.3, GII.4, GII.6, and GII.17, followed by full-length gene synthesis.

The synthesized gene fragments are ligated into a plasmid vector and the resulting recombinant plasmid vector is transformed into yeast by electroporation. The corresponding recombinant strains expressing norovirus antigens are obtained through screening and amplification. Here, the plasmid vector and yeast may be any plasmid vector and yeast commonly used in the art. For example, the plasmid vector is the pMAUR (S.C) KARS1 plasmid, and the yeast is *Hansenula polymorpha.*

The recombinant strains expressing norovirus antigens are activated and expanded, and the recombinant proteins are induced to be expressed in the plateau phase.

### (2) Purification of norovirus antigen proteins

The recombinant strains expressing the norovirus antigens are each resuspended in a Tris buffer system, such as a Tris buffer system at a concentration of 0.05 M to 1.5 M, to prepare a cell suspension. The cell suspension is centrifuged to harvest the cells, which are subsequently resuspended and lysed. The protein-containing supernatant is collected and subjected to column chromatography and gradient elution to collect the target proteins, thereby obtaining virus-like particles of various norovirus genotypes.

### (3) Mixing of norovirus antigens

The virus-like particles of norovirus genotypes GI.1, GII.2, GII.3, GII.4, GII.6, and GII.17 are separately added to a buffer comprising sodium chloride, and then the buffers comprising sodium chloride for the respective virus-like particles are mixed to obtain a norovirus antigen mixture solution.

### (4) Mixing and adsorption of norovirus antigen mixture solution with an adjuvant

The norovirus antigen mixture solution is added to an aluminum adjuvant and shaken for adsorption for 0.5-20 hours, thereby obtaining the hexavalent norovirus immunogenic composition according to the present invention.

### Immunoassay Kit

The immunoassay kit of the present invention comprises the hexavalent norovirus immunogenic composition of the present invention.

Preferably, the immunoassay kit is a kit for detecting norovirus.

The immunoassay kit of the present invention may further comprise an instruction manual, which contains a clear description of techniques to be employed when using the components in the kit to achieve desired results, such as detection results. Optionally, the immunoassay kit of the present invention may further comprise other applicable components, such as measuring tools, syringes, or other applicable accessories readily identifiable by those skilled in the art.

### Uses

The hexavalent norovirus immunogenic composition or the immunoassay kit of the present invention may be used for a variety of purposes, for example, for preventing and/or treating norovirus infection, for diagnosing norovirus infection, or as an immunogen for developing norovirus antibodies. The norovirus infection refers to gastroenteritis, in particular, acute viral gastroenteritis.

### Examples

The following examples are merely intended to illustrate the present invention, and not to limit the scope of the present invention.

All experimental methods used in the following examples are conventional experimental methods in the art, unless otherwise specified. All experimental materials used in the following examples are purchased from biochemical reagent suppliers, unless otherwise specified.

### Example 1: Preparation of Hexavalent Norovirus Immunogenic composition

(1) The virus-like particle (VLP) proteins of the following various norovirus genotypes were prepared separately, and their amino acid sequences thereof are set forth below:
   The VLP protein of norovirus genotype GI.1 having an amino acid sequence as set forth in SEQ ID NO: 1.
   The VLP protein of norovirus genotype GII.2 having an amino acid sequence as set forth in SEQ ID NO: 2.
   The VLP protein of norovirus genotype GII.3 having an amino acid sequence as set forth in SEQ ID NO: 3.
   The VLP protein of norovirus genotype GII.4 having an amino acid sequence as set forth in SEQ ID NO: 4.
   The VLP protein of norovirus genotype GII.6 having an amino acid sequence as set forth in SEQ ID NO: 5.
   The VLP protein of norovirus genotype GII.17 having an amino acid sequence as set forth in SEQ ID NO: 6.

The specific preparation method was as follows:
Codon optimization was performed on the gene sequences corresponding to the virus-like particle proteins of norovirus genotypes GI.1, GII.2, GII.3, GII.4, GII.6, and GII.17, respectively, followed by full-length gene synthesis, which was commissioned by Tsingke Biotechnology Co., Ltd. The synthesized target genes were ligated into the pMAUR (S.C) KARS1 expression vector via Gibson assembly, and the resulting recombinant vector was then transformed into *Hansenula polymorpha* by electroporation. Positive transformants were selected using G418 resistance, and large-scale cultivation was carried out to obtain recombinant strains expressing norovirus antigens.

The recombinant strains expressing norovirus antigens were inoculated into the yeast extract peptone dextrose medium at an inoculum size of 1% and cultured for 24 h for small-scale shaking activation. The activated strains were transferred into 50 mL of the yeast extract peptone dextrose medium for expanded culture in a horizontal shaking incubator. After culture at 30°C for 16 h until the strains reached the plateau phase, methanol was added to the medium for induction culture. Methanol addition was performed once every 24 h for a total of twice. At the end of induction, the cells were collected, lysed, and purified to obtain the proteins.

### (2) Purification of norovirus antigen proteins

The cells were resuspended in a 0.05 M Tris buffer system to prepare a cell suspension. After centrifugation for 3 h, the cells were collected, resuspended, and lysed using a low-temperature high-pressure homogenizer. The protein-containing supernatant was collected, subjected to column chromatography, and eluted using a 0.05 M Tris buffer containing salt to collect the target proteins.

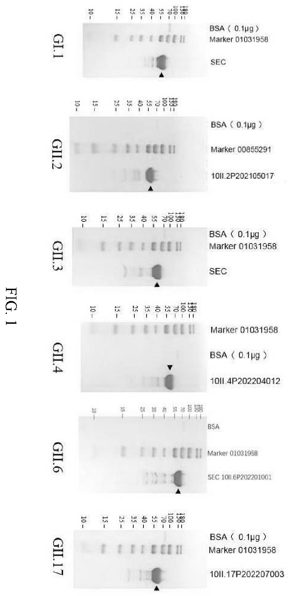

The protein purity was determined by vertical gel electrophoresis SDS-PAGE. The results are shown in FIG. 1, where the bands corresponding to the virus-like particles of each genotype are indicated by arrows. As can be seen from FIG. 1, effective purification of the virus-like particles of each genotype was achieved in this step.

### (3) Mixing and adsorption of norovirus antigens with an adjuvant

The virus-like particles of norovirus genotypes GI.1, GII.2, GII.3, GII.4, GII.6, and GII.17 were separately added to a buffer containing sodium chloride, and mixed uniformly at a weight ratio of 40: 20: 40: 40: 40: 40 for the virus-like particles of norovirus genotypes GI.1, GII.2, GII.3, GII.4, GII.6, and GII.17 to obtain a norovirus antigen mixture solution.

### (4) Mixing and adsorption of norovirus antigen mixture solution with an adjuvant

The norovirus antigen mixture solution was added to an aluminum hydroxide solution and shaken for adsorption for 2 hours.

Sample S1 was obtained, in which the concentrations of the virus-like particles of norovirus genotypes GI.1, GII.2, GII.3, GII.4, GII.6, and GII.17 were 40 µg/mL, 20 µg/mL, 40 µg/mL, 40 µg/mL, 40 µg/mL, and 40 µg/mL, respectively, and the concentration of aluminum hydroxide was 1.5 mg/mL. The details are shown in Table 1.

### Example 2: Preparation of Hexavalent Norovirus Immunogenic Composition

The specific preparation method of Example 2 was substantially the same as that of Example 1, with the only difference being that in Step (3), the virus-like particles of various norovirus genotype were uniformly mixed at the weight ratios shown in Table 1 to obtain Samples S2, S3, S4, S5, S6, S19, and S20.

In the obtained Sample S2, the concentrations of the virus-like particles of norovirus genotypes GI.1, GII.2, GII.3, GII.4, GII.6, and GII.17 were 40 µg/mL, 40 µg/mL, 40 µg/mL, 40 µg/mL, 40 µg/mL, and 40 µg/mL, respectively, and the concentration of aluminum hydroxide was 1.5 mg/mL. In the obtained Sample S3, the concentrations of the virus-like particles of norovirus genotypes GI.1, GII.2, GII.3, GII.4, GII.6, and GII.17 were 40 µg/mL, 20 µg/mL, 10 µg/mL, 40 µg/mL, 40 µg/mL, and 40 µg/mL, respectively, and the concentration of aluminum hydroxide was 1.5 mg/mL. In the obtained Sample S4, the concentrations of the virus-like particles of norovirus genotypes GI.1, GII.2, GII.3, GII.4, GII.6, and GII.17 were 40 µg/mL, 20 µg/mL, 20 µg/mL, 40 µg/mL, 40 µg/mL, and 40 µg/mL, respectively, and the concentration of aluminum hydroxide was 1.5 mg/mL. In the obtained Sample S5, the concentrations of the virus-like particles of norovirus genotypes GI.1, GII.2, GII.3, GII.4, GII.6, and GII.17 were 40 µg/mL, 20 µg/mL, 30 µg/mL, 40 µg/mL, 40 µg/mL, and 40 µg/mL, respectively, and the concentration of aluminum hydroxide was 1.5 mg/mL. In the obtained Sample S6, the concentrations of the virus-like particles of norovirus genotypes GI.1, GII.2, GII.3, GII.4, GII.6, and GII.17 were 30 µg/mL, 20 µg/mL, 40 µg/mL, 40 µg/mL, 40 µg/mL, and 40 µg/mL, respectively, and the concentration of aluminum hydroxide was 1.5 mg/mL. In the obtained Sample S19, the concentrations of the virus-like particles of norovirus genotypes GI.1, GII.2, GII.3, GII.4, GII.6, and GII.17 were 20 µg/mL, 20 µg/mL, 40 µg/mL, 40 µg/mL, 40 µg/mL, and 40 µg/mL, respectively, and the concentration of aluminum hydroxide was 1.5 mg/mL. In the obtained Sample S20, the concentrations of the virus-like particles of norovirus genotypes GI.1, GII.2, GII.3, GII.4, GII.6, and GII.17 were 10 µg/mL, 20 µg/mL, 40 µg/mL, 40 µg/mL, 40 µg/mL, and 40 µg/mL, respectively, and the concentration of aluminum hydroxide was 1.5 mg/mL.

The details are shown in Table 1.

### Comparative Example 1: Preparation of Monovalent Formulations

Monovalent formulations of genotypes GI.1, GII.2, GII.3, GII.4, GII.6, and GII.17 were prepared, corresponding to Samples S7, S8, S9, S10, S11, and S12, respectively.

The specific preparation method was substantially the same as that of Example 1, with the only differences being that in Step (3), the virus-like particles of various genotypes were not mixed; and in Step (4), the monovalent virus-like particles were subjected to mixing and adsorption in an aluminum hydroxide solution.

In Samples S7, S8, S9, S10, S11, and S12, the concentration of aluminum hydroxide was 1.5 mg/mL, and the protein contents were 40 µg/mL, 20 µg/mL, 40 µg/mL, 40 µg/mL, 40 µg/mL, and 40 µg/mL, respectively. The details are shown in Table 1.

### Comparative Example 2: Preparation of Comparative Samples for Hexavalent Norovirus Immunogenic Composition

In Comparative Example 2, the preparation method for the virus-like particles of norovirus genotypes GII.2, GII.4, GII.6, and GII.17 was the same as that in Example 1.

In Comparative Example 2, the sequence information of the virus-like particles of norovirus genotypes GI.1 and GII.3 is as follows:
The reference sequence 1 for the VLP protein of norovirus genotype GI.1 (hereinafter referred to as GI.1 Ref. 1) having an amino acid sequence as set forth in SEQ ID NO: 7.

The reference sequence 2 of the VLP protein of norovirus genotype GI.1 (hereinafter referred to as GI.1 Ref. 2) having an amino acid sequence as set forth in SEQ ID NO: 8.

The reference sequence 1 of the VLP protein of norovirus genotype GII.3 (hereinafter referred to as GII.3 Ref. 1) having an amino acid sequence as set forth in SEQ ID NO: 9, which corresponds to the amino acid sequence SEQ ID NO: 6 for GII.3 in Patent Application No. 202110861640.1.

The reference sequence 2 of the VLP protein of norovirus genotype GII.3 (hereinafter referred to as GII.3 Ref. 2) having an amino acid sequence as set forth in SEQ ID NO: 10.

The reference sequence 3 of the VLP protein of norovirus genotype GII.3 (hereinafter referred to as GII.3 Ref. 3) having an amino acid sequence as set forth in SEQ ID NO: 11.

The preparation method of the comparative samples for the hexavalent norovirus immunogenic composition in Comparative Example 2 was substantially the same as that in Example 1, with the only difference being that the corresponding virus-like particles of norovirus genotypes GI.1 or GII.3 were used in accordance with Table 1. Samples S13, S14, S15, S16, and S17 were obtained.

In Sample S13, the concentrations of the virus-like particles of norovirus genotypes GI.1 Ref. 1, GII.2, GII.3, GII.4, GII.6, and GII.17 were 40 µg/mL, 20 µg/mL, 40 µg/mL, 40 µg/mL, 40 µg/mL, and 40 µg/mL, respectively, and the concentration of aluminum hydroxide was 1.5 mg/mL. In Sample S14, the concentrations of the virus-like particles of norovirus genotypes GI.1 Ref. 2, GII.2, GII.3, GII.4, GII.6, and GII.17 were 40 µg/mL, 20 µg/mL, 40 µg/mL, 40 µg/mL, 40 µg/mL, and 40 µg/mL, respectively, and the concentration of aluminum hydroxide was 1.5 mg/mL. In Sample S15, the concentrations of the virus-like particles of norovirus genotypes GI.1, GII.2, GII.3 Ref. 1, GII.4, GII.6, and GII.17 were 40 µg/mL, 20 µg/mL, 40 µg/mL, 40 µg/mL, 40 µg/mL, and 40 µg/mL, respectively, and the concentration of aluminum hydroxide was 1.5 mg/mL. In Sample S16, the concentrations of the virus-like particles of norovirus genotypes GI.1, GII.2, GII.3 Ref. 2, GII.4, GII.6, and GII.17 were 40 µg/mL, 20 µg/mL, 40 µg/mL, 40 µg/mL, 40 µg/mL, and 40 µg/mL, respectively, and the concentration of aluminum hydroxide was 1.5 mg/mL. In Sample S17, the concentrations of the virus-like particles of norovirus genotypes GI.1, GII.2, GII.3 Ref. 3, GII.4, GII.6, and GII.17 were 40 µg/mL, 20 µg/mL, 40 µg/mL, 40 µg/mL, 40 µg/mL, and 40 µg/mL, respectively, and the concentration of aluminum hydroxide was 1.5 mg/mL. The details are shown in Table 1.

### Comparative Example 3: Preparation of Comparative Samples for Hexavalent Norovirus Immunogenic composition

A hexavalent norovirus vaccine was prepared in accordance with the method described in the "Specific Embodiments" section of the Chinese Patent Application No. 202110861640.1, to obtain Sample S18.

**Table 1**

| Sample | Type of virus-like particle | Protein content | Al(OH)₃ |
|---|---|---|---|
| No. | | (µg/mL) | concentration (mg/mL) |
| S1 | GI.1: GII.2: GII.3: GII.4: GII.6: GII.17 | 40: 20: 40: 40: 40: 40 | 1.5 |
| S2 | | 40: 40: 40: 40: 40: 40 | 1.5 |
| S3 | | 40: 20: 10: 40: 40: 40 | 1.5 |
| S4 | | 40: 20: 20: 40: 40: 40 | 1.5 |
| S5 | | 40: 20: 30: 40: 40: 40 | 1.5 |
| S6 | | 30: 20: 40: 40: 40: 40 | 1.5 |
| S19 | | 20: 20: 40: 40: 40: 40 | 1.5 |
| S20 | | 10: 20: 40: 40: 40: 40 | 1.5 |
| S7 | GI.1 | 40 | 1.5 |
| S8 | GII.2 | 20 | 1.5 |
| S9 | GII.3 | 40 | 1.5 |
| S10 | GII.4 | 40 | 1.5 |
| S11 | GII.6 | 40 | 1.5 |
| S12 | GII.17 | 40 | 1.5 |
| S13 | GI.1 Ref.1: GII.2: GII.3: GII.4: GII.6: GII.17 | 40: 20: 40: 40: 40: 40 | 1.5 |
| S14 | GI.1 Ref. 2: GII.2: GII.3: GII.4: GII.6: GII.17 | 40: 20: 40: 40: 40: 40 | 1.5 |
| S15 | GI.1: GII.2: GII.3 Ref.1: GII.4: GII.6: GII.17 | 40: 20: 40: 40: 40: 40 | 1.5 |
| S16 | GI.1: GII.2: GII.3 Ref.2: GII.4: GII.6: GII.17 | 40: 20: 40: 40: 0: 40 | 1.5 |
| S17 | GI.1: GII.2: GII.3 Ref.3: GII.4: GII.6: GII.17 | 40: 20: 40: 40: 40: 40 | 1.5 |
| S18 | Hexavalent norovirus vaccine prepared in accordance with the method described in the "Specific Embodiments" section of the Chinese Patent Application No. 202110861640.1 | | |

### Experimental Example 1: Screening and Efficacy Evaluation of Hexavalent Norovirus Immunogenic Composition

(1) Experimental animals: Female 6-week-old BALB/c mice, n = 104, purchased from Shanghai Lingchang Biotechnology Co., Ltd., with the animal license No. SCXK(Hu) 2018-0003.
(2) Animal grouping: The mice were randomly divided into 13 groups, with 8 mice per group, and assigned to test Sample S1, the aluminum hydroxide adjuvant group (at a concentration of 1.5 mg/mL), Samples S7 to S12, and Samples S13 to S18, respectively.
(3) Animal immunization: Intramuscular immunization was adopted, with a dose of 100 µL per mouse for each group. Each sample was administered three times via repeated injection at wk00, wk03, and wk06. Blood sampling was performed at wk05, wk08, wk10, and wk14. During the experiment, specific IgG antibody titers were determined.

### (4) Detection method:

The indirect method was used to determine the levels of specific IgG antibodies in serum samples. Purified VLPs were coated onto 96-well ELISA plates to form solid-phase antigens. After blocking treatment, the sera to be tested were serially diluted starting from a conventional initial dilution, and multiple dilutions were prepared. The serially diluted serum samples were added to the 96-well ELISA plates, and then bound with HRP-labeled anti-IgG/IgG1/IgG2a antibodies to form antigen-antibody (serum)-enzyme-labeled antibody complexes. Finally, the TMB substrate was added for color development, and the absorbance (OD value) at 450 nm was measured using a microplate reader. The developed color intensities were positively correlated with the levels of specific IgG/IgG1/IgG2a antibodies in the test samples. The antibody titers were determined by fitting a correlation curve to the absorbance (OD value) versus the dilution factor (Log) of the serum samples.

(5) Determination method (two-way ANOVA of Log2 values followed by Tukey's multiple comparison analysis): For the sigmoidal gradient curve of the antigen-antibody reaction, the upper limit of the OD value was set to approximately 2.0 and the lower limit to approximately 0.2. The logarithmic linear segment was used to calculate the abscissa value when the ordinate was 0, namely the endpoint titers of specific antibodies in serum samples. In cases where the OD value at the initial dilution was too low to plot the sigmoidal gradient curve of the antigen-antibody reaction, the titers were determined as 10 for all samples with OD < 0.1, and the titers were determined as the initial dilution for all samples with 0.1 ≤ OD ≤ 0.2.

### (6) Detection results

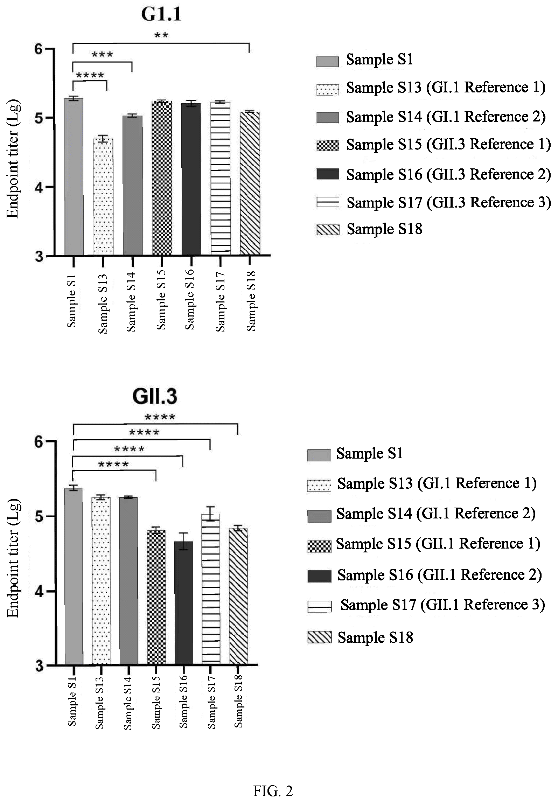

FIG. 2 shows the serum IgG antibody levels (95% CI) in mice from the hexavalent formulation group (Sample S1) and the comparative sample groups (i.e., Samples S13 to S18) at Week 14 (wk14) in this experimental example.

The IgG antibody titers (Lg) against genotypes GI.1, GII.2, GII.3, GII.4, GII.6, and GII.17 induced by the hexavalent norovirus immunogenic composition prepared with Sample S1 were the highest, with values of 5.28, 5.00, 5.38, 4.63, 4.93, and 5.44, respectively. Compared with the hexavalent compositions prepared with different GI.1 antigens (i.e., Samples S13 and S14), the IgG antibody titer against GI.1 for Sample S13 was 4.70, which was significantly lower than that for Sample S1 (5.28). The IgG antibody titer of GI.1 for Sample S14 was 5.031, which was slightly higher than that for Sample S13, but significantly lower than that for Sample S1 (5.28). Therefore, Sample S1 exhibited the highest IgG antibody titer against GI.1 with significant differences, indicating that GI.1 in Sample S1 had a lower interference with other genotypes and a favorable compatibility.

Compared with the hexavalent compositions prepared with different GII.3 antigens (i.e., Samples S15, S16, and S17), the IgG antibody titers against GII.3 for Samples S15, S16, and S17 were 4.81, 4.66, and 5.02, respectively. The titer for Sample S16 was the lowest, while those for Samples S15 and S17 were slightly higher, all of which were significantly lower than that for Sample S1 (5.38). Therefore, Sample S1 exhibited the highest IgG antibody titer against GII.3 with significant differences, indicating that GII.3 in Sample S1 had a lower interference with other genotypes and a favorable compatibility.

GI.1 virus-like particles (having the amino acid sequence as set forth in SEQ ID NO: 1) and GII.3 virus-like particles (having the amino acid sequence as set forth in SEQ ID NO: 3) with better anti-interference capability were screened and used to form a hexavalent norovirus immunogenic composition (i.e., Sample S1). Compared with the immune effect of Sample S18, the IgG antibody titer against GII.3 (5.38) was significantly higher than that of Sample S18 (4.83) with a significant difference, and the IgG antibody titer against GI.1 (5.28) was also significantly higher than that of Sample S18 (5.08) with a significant difference. For the other genotypes, namely GII.2, GII.4, GII.6, and GII.17, slightly better immune effects were also observed compared with those of Sample S18. After comprehensive consideration, genotypes GI.1 and GII.3 with better anti-interference capability were screened and combined with GII.2, GII.4, GII.6, and GII.17 to form the hexavalent norovirus immunogenic composition (Sample S1), in which the interference among the different genotypes was minimal, demonstrating optimal compatibility.

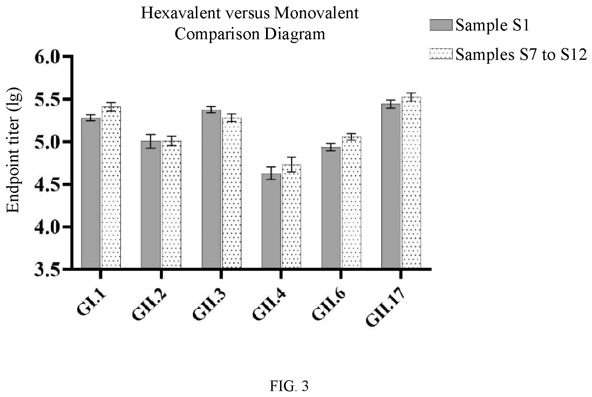

In addition, FIG. 3 shows the serum IgG antibody levels (95% CI) in mice from the hexavalent formulation group (Sample S1) and the monovalent formulation groups (Samples S7 to S12) at Week 14 (wk14) in this experimental example. The IgG antibody titers (Lg) induced by the various monovalent formulations (i.e., Samples S7 to S12) corresponding to GI.1, GII.2, GII.3, GII.4, GII.6, and GII.17 contained in Sample S1 were 5.41, 5.00, 5.28, 4.73, 5.05, and 5.52, respectively. High levels of IgG antibodies were maintained at 8 weeks after three administrations (wk14), with the IgG antibody titers (Lg) remaining above 4.50.

Compared with the corresponding monovalent formulations, the IgG antibody levels induced by the hexavalent norovirus immunogenic composition (Sample S1) tended to be consistent with those of the corresponding monovalent formulations, and the IgG antibody titers were substantially equivalent to those induced by each monovalent formulation, with no significant difference (P < 0.05), indicating favorable mutual compatibility among the antigens in the hexavalent norovirus immunogenic composition. That is, there was almost no mutual interference among the different antigens in the hexavalent norovirus immunogenic composition. After combining GII.2, GII.4, GII.6, and GII.17 with GI.1 and GII.3 to form the hexavalent norovirus immunogenic composition, the mutual compatibility of the various antigens was greatly improved; the interference among the different antigens was reduced, and an unexpected synergistic effect occurred, leading to a significant immune effect.

Experimental Example 2: Screening of Antigen Ratios for Hexavalent Norovirus Immunogenic composition
(1) Experimental animals: Female BALB/c mice, 6-week-old, n = 48, purchased from Shanghai Lingchang Biotechnology Co., Ltd., with the animal license No. SCXK(Hu) 2018-0003.
(2) Animal grouping: The mice were randomly divided into 8 groups, with 6 mice per group, and assigned to test Samples S1 to S6, S19, and S20, respectively.
(3) Animal immunization: Intramuscular immunization was adopted, with a dose of 100 µL per mouse for each group. Each sample was administered three times via repeated injection at wk00, wk03, and wk06. Blood sampling was performed at wk05, wk06, or wk08. During the experiment, blocking antibody titers were determined.
(4) Detection method for blocking antibody titers in serum

The sera to be tested were serially diluted with a diluent starting from a conventional initial dilution to prepare multiple dilutions. Biotin-labeled human histo-blood group antigen (HBGA) was sufficiently bounded onto streptavidin microplates. Meanwhile, the diluted serum samples were mixed with an equal volume of purified VLP working solution and incubated. The HBGA-bound streptavidin microplates were washed. The incubated VLP-serum mixtures were added to the microplates and incubated, followed by washing plate. Rabbit polyclonal antibody was added, followed by incubation, and the plates were washed again. HRP-labeled goat anti-rabbit IgG was added to form antigen-antibody (serum)-enzyme-labeled antibody complexes. Finally, the TMB substrate was added for color development, and the absorbance (OD value) at 450 nm was measured using a microplate reader.

(5) Evaluation indices: Blocking index = (1 - OD value of serum group / OD value of positive control) × 100%. The BT50 value was calculated, and defined as the highest serum dilution capable of blocking 50% of the binding between VLPs and HBGA.

### (6) Detection results

The serum GMBT50 values (95% CI) determined at two weeks after the third immunization (wk08) were as follows: for Sample S1 (4/2/4/4/4/4), the serum GMBT50 values against the hexavalent composition containing GI.1, GII.2, GII.3, GII.4, GII.6, and GII.17 were 720, 1524, 640, 679, 539, and 1210, respectively, indicating an optimal immune effect.

For the immune effect against GII.2 of the hexavalent composition Sample S2 (4/4/4/4/4/4), the GMBT50 value against GII.2 was 1440, which was slightly lower than that of Sample S1 (1524), with no significant difference. Increasing the proportion of GII.2 reduced the immune effect against GII.2 instead. Therefore, the hexavalent composition Sample S2 (4/4/4/4/4/4) exhibited a poorer immune effect.

In the weight ratio screening for GII.3, different weight ratios had different impacts on the immune effect. The GMBT50 values against GII.3 for Sample S3 (4/2/1/4/4/4), Sample S4 (4/2/2/4/4/4), and Sample S5 (4/2/3/4/4/4) were 400, 480, and 600, respectively. Compared with the GMBT50 value of Sample S1 (640), Sample S3 exhibited the poorest immune effect, followed by Sample S4, with significant differences. The GMBT50 value of Sample S5 was slightly lower than that of Sample S1, with no significant difference. Furthermore, the weight ratio screening for GII.3, Samples S3, S4, and S5 also affected the immune effect against GII.2, correspondingly lowering their GMBT50 values against GII.2 simultaneously. In addition, the weight ratio screening for GII.3, Samples S3, S4, and S5 had little effect on the immune effects against GI.1, GII.4, GII.6, and GII.17, and their respective GMBT50 values were essentially identical to those of Sample S1.

In the weight ratio screening for GI.1, different weight ratios had different impacts on the immune effect. The GMBT50 values against GI.1 for Sample S6 (3/2/4/4/4/4), Sample S19 (2/2/4/4/4/4), and Sample S20 (1/2/4/4/4/4) were 560, 440, and 400, respectively. Compared with the GMBT50 value of Sample S1 (720), Sample S20 exhibited the poorest immune effect, followed by Sample S19; Sample S6 exhibited a slightly better immune effect, with a value slightly lower than that of Sample S1, and all differences were statistically significant. In addition, in the weight ratio screening for GI.1, Samples S6, S19, and S20 had little effects on the immune effects against GII.2, GII.3, GII.4, GII.6, and GII.17, which were slightly lower than that of Sample S1, with no significant difference.

Therefore, compared with Samples S2, S3, S4, S5, S6, S19, and S20, Sample S1, which contains the six genotypes GI.1, GII.2, GII.3, GII.4, GII.6, and GII.17, exhibited the highest GMBT50 values, and the immune effect of Sample S5 was slightly lower than that of Sample S1, indicating that when the weight ratio of the various genotypes is 4/2/4/4/4/4, the interference among them is minimized and the compatibility is optimal. In other words, this ratio significantly reduces the interference among the various genotypes, further improves their compatibility, and enhances the immune effect.

The above description is merely several exemplary embodiments of the present invention, and shall not impose any limitation on the present invention in any form. Although the present invention has been disclosed above with reference to the preferred examples, it is not intended to limit the present invention. Any identical or equivalent examples obtained by any technical personnel familiar with the pertinent art through making minor modifications or alterations to the technical contents disclosed above, without departing from the scope of the technical solutions of the present invention, shall fall within the scope of the present invention.

## Claims

1. A hexavalent norovirus immunogenic composition, **characterized in that** it comprises virus-like particles of norovirus genotypes GI.1, GII.2, GII.3, GII.4, GII.6, and GII.17, or active fragments thereof.

2. The hexavalent norovirus immunogenic composition according to claim 1, **characterized in that** the virus-like particle of norovirus genotype GI.1 or active fragment thereof comprises or consists of an amino acid sequence selected from the group consisting of:
(1) an amino acid sequence as set forth in SEQ ID NO: 1;
(2) an amino acid sequence having at least 90%, 91%, 92%, 93%, 94%, 95%, 96%, 97%, 98%, 99%, or more identity to the amino acid sequence as set forth in SEQ ID NO: 1; and
(3) an amino acid sequence having one or more amino acid substitutions, deletions, or insertions relative to the amino acid sequence as set forth in SEQ ID NO: 1;
preferably, the amino acid sequence of the virus-like particle of norovirus genotype GI.1 or active fragment thereof is as set forth in SEQ ID NO: 1.

3. The hexavalent norovirus immunogenic composition according to claim 1 or 2, **characterized in that** the virus-like particle of norovirus genotype GII.2 or active fragment thereof comprises or consists of an amino acid sequence selected from the group consisting of:
(1) an amino acid sequence as set forth in SEQ ID NO: 2;
(2) an amino acid sequence having at least 90%, 91%, 92%, 93%, 94%, 95%, 96%, 97%, 98%, 99%, or more identity to the amino acid sequence as set forth in SEQ ID NO: 2; and
(3) an amino acid sequence having one or more amino acid substitutions, deletions, or insertions relative to the amino acid sequence as set forth in SEQ ID NO: 2;
preferably, the amino acid sequence of the virus-like particle of norovirus genotype GII.2 or active fragment thereof is as set forth in SEQ ID NO: 2.

4. The hexavalent norovirus immunogenic composition according to any one of claims 1 to 3, **characterized in that** the virus-like particle of norovirus genotype GII.3 or active fragment thereof comprises or consists of an amino acid sequence selected from the group consisting of:
(1) an amino acid sequence as set forth in SEQ ID NO: 3;
(2) an amino acid sequence having at least 90%, 91%, 92%, 93%, 94%, 95%, 96%, 97%, 98%, 99%, or more identity to the amino acid sequence as set forth in SEQ ID NO: 3; and
(3) an amino acid sequence having one or more amino acid substitutions, deletions, or insertions relative to the amino acid sequence as set forth in SEQ ID NO: 3;
preferably, the amino acid sequence of the virus-like particle of norovirus genotype GII.3 or active fragment thereof is as set forth in SEQ ID NO: 3.

5. The hexavalent norovirus immunogenic composition according to any one of claims 1 to 4, **characterized in that** the virus-like particle of norovirus genotype GII.4 or active fragment thereof comprises or consists of an amino acid sequence selected from the group consisting of:
(1) an amino acid sequence as set forth in SEQ ID NO: 4;
(2) an amino acid sequence having at least 90%, 91%, 92%, 93%, 94%, 95%, 96%, 97%, 98%, 99%, or more identity to the amino acid sequence as set forth in SEQ ID NO: 4; and
(3) an amino acid sequence having one or more amino acid substitutions, deletions, or insertions relative to the amino acid sequence as set forth in SEQ ID NO: 4;
preferably, the amino acid sequence of the virus-like particle of norovirus genotype GII.4 or active fragment thereof is as set forth in SEQ ID NO: 4.

6. The hexavalent norovirus immunogenic composition according to any one of claims 1 to 5, **characterized in that** the virus-like particle of norovirus genotype GII.6 or active fragment thereof comprises or consists of an amino acid sequence selected from the group consisting of:
(1) an amino acid sequence as set forth in SEQ ID NO: 5;
(2) an amino acid sequence having at least 90%, 91%, 92%, 93%, 94%, 95%, 96%, 97%, 98%, 99%, or more identity to the amino acid sequence as set forth in SEQ ID NO: 5; and
(3) an amino acid sequence having one or more amino acid substitutions, deletions, or insertions relative to the amino acid sequence as set forth in SEQ ID NO: 5;
preferably, the amino acid sequence of the virus-like particle of norovirus genotype GII.6 or active fragment thereof is as set forth in SEQ ID NO: 5.

7. The hexavalent norovirus immunogenic composition according to any one of claims 1 to 6, **characterized in that** the virus-like particle of norovirus genotype GII.17 or active fragment thereof comprises or consists of an amino acid sequence selected from the group consisting of:
(1) an amino acid sequence as set forth in SEQ ID NO: 6;
(2) an amino acid sequence having at least 90%, 91%, 92%, 93%, 94%, 95%, 96%, 97%, 98%, 99%, or more identity to the amino acid sequence as set forth in SEQ ID NO: 6; and
(3) an amino acid sequence having one or more amino acid substitutions, deletions, or insertions relative to the amino acid sequence as set forth in SEQ ID NO: 6;
preferably, the amino acid sequence of the virus-like particle of norovirus genotype GII.17 or active fragment thereof is as set forth in SEQ ID NO: 6.

8. The hexavalent norovirus immunogenic composition according to any one of claims 1 to 7, **characterized in that** the weight ratio of the virus-like particles of norovirus genotypes GI.1, GII.2, GII.3, GII.4, GII.6, and GII.17 or active fragments thereof is 40: 20: (10~100): 40: 40: 40, preferably 40: 20: (30~40): 40: 40: 40.

9. The hexavalent norovirus immunogenic composition according to any one of claims 1 to 8,
**characterized in that** it further comprises an adjuvant, preferably an aluminum adjuvant;
optionally, it further comprises sodium chloride;
optionally, it further comprises water.

10. An immunoassay kit, **characterized in that** it comprises the hexavalent norovirus immunogenic composition according to any one of claims 1 to 9;
preferably, the immunoassay kit is a kit for detecting norovirus.

11. Use of the hexavalent norovirus immunogenic composition according to any one of claims 1 to 9 or the immunoassay kit according to claim 10 in the manufacture of the following products:
(1) a medicament for preventing and/or treating norovirus infection;
(2) a kit for diagnosing norovirus infection; or
(3) an immunogen for developing norovirus antibodies;
preferably, the norovirus infection is gastroenteritis; more preferably, the norovirus infection is acute viral gastroenteritis.
